# EUROPÄISCHE PATENTSCHRIFT

(11) **EP 0 819 235 B1**
(45) Veröffentlichungstag und Bekanntmachung des Hinweises auf die Patenterteilung: **21.07.1999**
(21) Anmeldenummer: 96914886.5
(22) Anmeldetag: 09.04.1996
(51) Int. Cl.: F24F 3/12

(54) **VERFAHREN ZUM BEDUFTEN VON RÄUMEN UND RAUMBEDUFTUNGSGERÄT**
METHOD OF FRESHENING AIR IN ROOMS AND AN AIR-FRESHENER DEVICE
PROCEDE PERMETTANT DE DIFFUSER UN PARFUM DANS DES LOCAUX ET APPAREIL PREVU A CET EFFET

(30) Priorität: 07.04.1995 DE 29505589 U
(43) Veröffentlichungstag der Anmeldung: 21.01.1998
(73) Patentinhaber: Doerfel, Friedrich, 31303 Burgdorf (DE)
(72) Erfinder: Doerfel, Friedrich, D-31303 Burgdorf (DE); Mayer, Heinz, D-73271 Holzmaden (DE)
(74) Vertreter: Schumacher, Horst, Dr. Dipl.-Phys.
(86) Internationale Anmeldenummer: EP9601507
(87) Internationale Veröffentlichungsnummer: WO9631741

(56) Entgegenhaltungen:
- EP-A- 0 345 149
- FR-A- 772 069
- US-A- 5 114 625
- US-A- 5 302 359

## Beschreibung

Die Erfindung betrifft ein Raumbeduftungsgerät mit einem Luftleitkanal, mit einem Gebläse zum Fördern von zu beduftender Luft durch den Luftleitkanal, mit einem Vorratsbehälter für einen den Duftstoff beinhaltenden Flüssigkeitsvorrat und mit einer Flüssigkeitsverdunstungsfläche, über die der zu beduftende Luftstrom zur Aufnahme von verdunstendem Duftstoff geleitet wird sowie ein dementsprechendes Verfahren.

Ein solches Gerät ist au der US-A-5 302 359 bekannt.

Es sind kleine Duftspender bekannt (siehe US-A-5 114 625), die in der Regel aus einem Vorratsgefäß oder Vorratsbehälter bestehen, die einen Duftstoff aufnehmen und diesen über einen Docht an die Raumluft abgeben. Diese Geräte sind nur für sehr kleinvolumige Räume geeignet und geben den Duftstoff ungesteuert und erst recht ungeregelt an die Raumluft ab.

Es sind auch sogenannte "Duftsäulen" bekannt, z. B. mit einer Höhe von 0,8 m und einem Durchmesser von etwa 0,14 m. Bei diesen wird der Duftstoff aus einem Vorratsbehälter mittels einer Schlauchpumpe in eine erhitzte Pfanne gepumpt. Der verdunstende Duftstoff wird von einem oberhalb dieser Pfanne installierten Gebläse angesaugt oder fortgeblasen. Mit diesem Gerät können Räume bis maximal etwa 1000 m³ beduftet werden. Sowohl die Zudosierung von Duftstoff bzw. eine den Duftstoff enthaltende Flüssigkeit in die Pfanne als auch eine für ein angenehmes Raumklima wichtige gleichmäßige und einstellbare Duftstoffaufnahme durch den zu beduftenden Luftstrom sind bei diesen Geräten wenig zufriedenstellend.

Davon ausgehend liegt der Erfindung die Aufgabe zugrunde, ein Raumbeduftungsgerät und ein Verfahren zum Beduften von Räumen der eingangsgenannten Art zu schaffen, welches eine gut steuerbare Duftstoffaufnahme durch den Luftstrom ermöglicht. Es ist ferner wünschenswert, einzelne oder eine Mehrzahl von Räumen mit relativ großem Luftvolumen, insbesondere von über 1000 m³ Raumvolumen beduften zu können, wobei eine kompakte Bauweise des Raumbeduftungsgerätes angestrebt wird.

Zur Lösung dieser Aufgabe wird hinsichtlich eines Raumbeduftungsgerätes der eingangsgenannten Art vorgeschlagen, daß der Vorratsbehälter eine nach unten weisende Entleerungsöffnung aufweist, durch welche die Flüssigkeit den Vorratsbehälter nach unten verläßt, um sich auf eine unter der Entleerungsöffnung gelegene, bezüglich der Entleerungsöffnung schräg nach unten geeignete Oberfläche eines Verdunstungskörpers (Schrägfläche) zu verteilen, daß ferner ein die Entleerungsöffnung beherrschendes Dosierventil zur dosierten Abgabe von Flüssigkeit aus dem Vorrat an die Schrägfläche vorgesehen ist, und daß die Schrägfläche innerhalb des Luftleitkanals derart angeordnet ist, daß der zu beduftende Luftstrom intensiv über die Schrägfläche streicht.

Durch die Erfindung wird unter anderem erreicht, daß die Dosierung der den Duftstoff beinhaltenden Flüssigkeit auf die darunterliegende Schrägfläche in vergleichsweise einfacher aber sehr präziser Weise in kleinen Abgaberaten möglich wird und gleichzeitig eine für große Luftströmungsraten geeignet große Flüssigkeitsverdunstungsfläche realisiert wird, auf der sich die zudosierte den Duftstoff enthaltende Flüssigkeit trotz kleiner Zudosierraten sehr gleichmäßig und in sehr dünner Schicht verteilt ohne daß Duftstoff vergeudet wird oder Flüssigkeitströpfchen von dem Luftstrom in unzulässigem Maße mitgerissen werden. Mithin wird durch die Erfindung auch hygienischen Gesichtspunkten Rechnung getragen, indem Staub aufnehmende Flüssigkeitsreservoire so gut wie vollständig vermieden werden. Der Duftstoff kann in dem Vorratsbehälter unter besonders günstigen Bedingungen aufbewahrt werden, ohne, daß Duftstoffe verloren gehen und/oder sich die Duftstoffkonzentration im Laufe der Zeit ändert. Es ist daher möglich, das Raumbeduftungsgerät bei minimalem Wartungsaufwand, insbesondere ohne viel Reinigungsarbeit auch kurzzeitig z. B. für nur wenige Stunden am Tag zu benutzen. Ebenso einfach gestaltet sich der Austausch der Vorratsbehälter, sei es, daß ein Vorratsbehälter leer ist und durch einen vollen ersetzt werden muß, oder sei es, daß ein noch nicht leerer Duftstoffbehälter gegen einen Duftstoffbehälter mit einer anderen Duftnote ausgetauscht werden soll, um später im Bedarfsfalle wieder den ersten weiterbenutzen zu können. Ein besonderer Vorteil besteht darin, daß aufwendige Förderorgane, wie Schlauchpumpen oder dergleichen völlig vermieden werden können, weil die Flüssigkeitsförderung aus dem Vorrat auf die Schrägfläche allein durch Gravitation erfolgen kann. Die Schrägstellung der Flüssigkeitsverdunstungsfläche bewirkt, daß auch bei sehr kleinen Dosierraten von z. B. 3,6 cm³ duftstoffenthaltender Flüssigkeit pro Stunde in sechs Dosiertakten, eine Feinverteilung der Flüssigkeit über eine relativ große und gut von einem starken Luftstrom anströmbare Verdunstungsoberfläche selbstätig verteilt wird, so daß das relativ kleinbauende Raumbeduftungsgerät, das in seinen Abmessungen den sogenannten Duftsäulen durchaus ähnlich ist, für einen zu beduftenden Luftstrom von z. B. 400 m³ pro Stunde geeignet ist. Es ist sogar möglich, ein derartiges, als "Stand-Alone-Gerät" konzipiertes Raumbeduftungsgerät über einen Luftsammelkanal mit dem Luftkanal einer Klimaanlage zu verbinden, indem die beduftete Luft eingespeist wird und sich dadurch auch auf entlegene Räume oder Räume in anderen Etagen in der gewünschten bzw. erforderlichen Duftstoffkonzentration gleichmäßig verteilen kann.

Ein erfindungsgemäßes Raumbeduftungsgerät sowie das dementsprechende Verfahren zum Beduften von Räumen kann nun auf verschiedenste Weise realisiert werden, was anhand konkreter Ausführungsbeispiele unter Bezugnahme auf die Zeichnungen nachfolgend näher erläutert wird.

Zweckmäßige Ausgestaltungen des Erfindungsgegenstandes, die insbesondere eine gute Dosierung und Verteilung des Duftstoffes, insbesondere eine gute Steuerbarkeit bzw. Regelbarkeit der Duftstoffabgabe ermöglichen bzw. unangenehme Begleiterscheinungen, wie Überkonzentrationen und hygienische Probleme zu vermeiden gestatten, sind in weiteren Ansprüchen enthalten.

Die vorgenannten, sowie die beanspruchten und in den Ausführungsbeispielen beschriebenen, erfindungsgemäß zu verwendenden Verfahrensschritte sowie Bauteile unterliegen hinsichtlich ihrer Verfahrensbedingungen, ihrer Größe, Formgestaltung, Materialauswahl und technischen Konzeption keinen besonderen Ausnahmebedingungen, so daß die in dem jeweiligen Anwendungsgebiet bekannten Auswahlkriterien uneingeschränkt Anwendung finden können.

Weitere Einzelheiten, Merkmale und Vorteile des Gegenstandes der Erfindung ergeben sich aus der nachfolgenden Beschreibung der zugehörigen Zeichnung, in der - beispielhaft - bevorzugte Ausführungsformen dargestellt sind. In der Zeichnung zeigen:
- Fig. 1: eine erste Ausführungsform eines Raumbeduftungsgerätes im Vertikalschnitt;
- Fig. 2: eine zweite Ausführungsform eines Raumbeduftungsgerätes wiederum im Vertikalschnitt;
- Fig. 3: von demselben Raumbeduftungsgerät ein Horizontalschnitt entlang der Linie III-III gemäß Fig. 2;
- Fig. 4: von demselben Raumbeduftungsgerät ein Horizontalschnitt entlang der Linie IV-IV gemäß Fig. 2;
- Fig. 5: von demselben Raumbeduftungsgerät eine vergrößerte Detaildarstellung eines Dosierventilstößels um Axialschnitt;
- Fig. 6: von demselben Dosierventilstößel eine Ansicht von oben (Ansicht A-A gemäß Fig. 5);
- Fig. 7A-C: von demselben Raumbeduftungsgerät ein Dosierventil im Axialschnitt in drei Arbeitspositionen (Fig. 7 A: Geschlossen-Stellung; Fig. 7B: Portionierstellung; Fig. 7C: Auslaß-Stellung);
- Fig. 8: eine dritte Ausführungsform eines Raumbeduftungsgerätes im Vertikalschnitt (Schnitt entlang der Linie VIII-VIII gemäß Fig. 9);
- Fig. 9: von demselben Raumbeduftungsgerät eine Ansicht von oben bei abgenommenem Deckel (Ansicht B-B gemäß Fig. 8);
- Fig. 10: eine vierte Ausführungsform eines Raumbeduftungsgerätes im Axialschnitt (Schnitt entlang der Linie X-X gemäß Fig. 11);
- Fig. 11: von demselben Raumbeduftungsgerät eine Ansicht von oben (Ansicht C-C gemäß Fig. 10) sowie
- Fig. 12: für das Raumbeduftungsgerät nach Fign. 2 bis 4 eine Anschlußvariante an einen Lüftungskanal - schematisch.

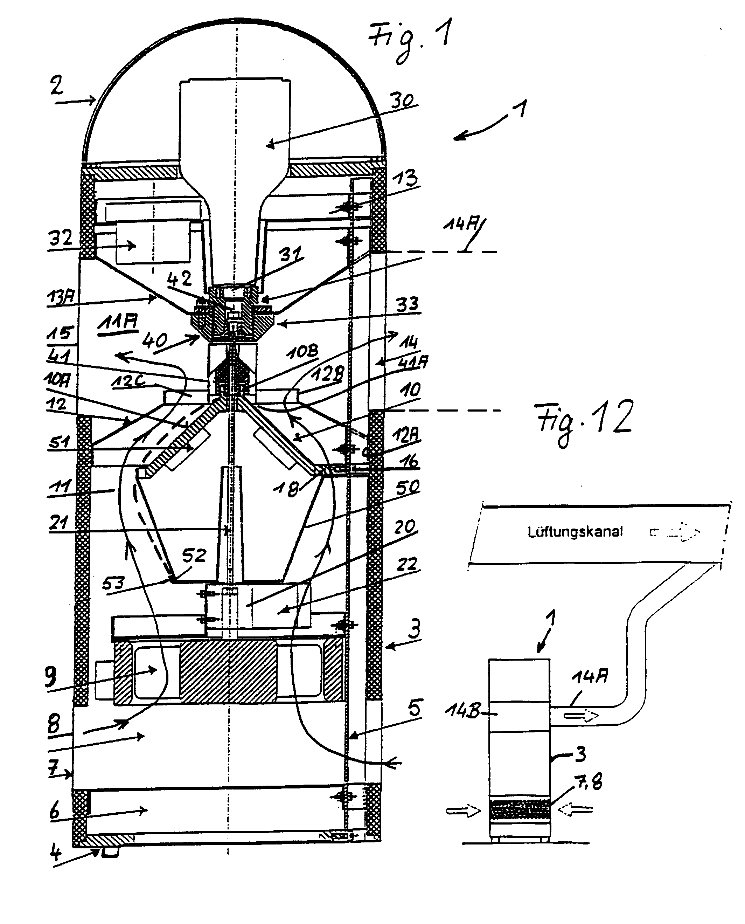

Wie aus Fig. 1 ersichtlich ist das Raumbeduftungsgerät 1 als Stand-Alone-Gerät in kreiszylindrischer Form mit halbkugeliger Haube 2 auf einen röhrenförmigen Gehäuse 3 gestaltet und mit Standfüßen 4 sowie drei an der Gehäuseinnenfläche achsparallel und gleichmäßig umfangsverteilt angeordneten Befestigungsstreben 5 versehen. Oberhalb eines Sockels 6 ist auf dem gesamten Gehäuseumfang ein ringförmiger Lufteinlaß 7 vorgesehen, der mit einer Loch- oder Gitterabdeckung 8 optisch gestaltet und gegen Eindringen gröberer Teile geschützt ist.

Unmittelbar oberhalb des Lufteinlasses 7 ist ein Gebläse 9 zur Erzeugung eines umfangsverteilten, nach oben gerichteten Luftstromes (durch Strömungspfeile dargestellt) angeordnet. Die Luftströmung umströmt einen hohlkegelförmigen koaxial mit seiner Spitze nach oben gerichteten und an den Befestigungsstreben gehaltenen Verdunstungskörper 10 über einen ringförmigen Strömungspalt 11 zwischen der Innenwandfläche des Gehäuses 3 und den radialen Außenflächen des kreiskegelförmigen Verdunstungshohlkörpers 10 sowie eines eng darunter sitzenden runden Auffangbehälters 50, die gemeinsam einen strömungsgünstigen, etwa tropfenförmigen Verdrängungskörper bilden. Die umströmten Flächen können auch (nach außen) bauchig geformt sein (siehe gestrichelte Linie in der linken Bildhälfte).

Ein hohlkegelförmiger Luftleitmantel 12 liegt mit einer unteren Schürze 12 A dicht am Gehäuse 3 an, während eine nach oben gerichtete Schürze 12 B eine zentrale Luftabströmöffnung 12 C begrenzt. Die Form und die Anordnung des Luftleitmantels 12 ist so gewählt, daß sich der Strömungsspalt 11 zwischen der radialen Außenfläche des Verdunstungskörpers 10 und der radialen Innenfläche des Luftleitmantels 12 nach oben hin leicht verjüngt, so daß ein Venturieffekt entsteht und der aufwärts getriebene Luftstrom konzentriert nach radial innen und oben mit zunehmender Geschwindigkeit eng an der radialen Außenfläche (Flüssigkeitsverdunstungsfläche 10 A) entlang geführt wird.

Der Luftstrom verbreitert sich nach seinem Austritt aus der Luftabströmöffnung 12 C sehr rasch durch Umlenkung nach radial außen unter gleichzeitiger vertikaler Ausweitung des Strömungsspaltes. Dabei dient die radiale Außenseite des Luftleitmantels 12 einerseits sowie die radiale Außenseite eines nach unten zulaufenden hohlkegelförmigen Vorratsraumbodens 13 A als seitliche Begrenzung eines Radialströmungskanales 11 A, der nach radial außen mit einem - ähnlich dem Lufteinlaß 7 gestalteten - kreisringförmigen Luftauslaß 14 mit einer Loch- oder Gitterabdeckung 15 - ähnlich der entsprechenden Abdeckung 8 begrenzt ist. Anstelle der Loch- oder Gitterabdeckung 15 kann eine nicht gelochte, flache oder ringkanalförmige Abdeckung mit einem Luftauslaßstutzen 14 A für den Anschluß an einen gebäudeeigenen Lüftungskanal vorgesehen sein (siehe Fig. 12).

Ein den Duftstoff enthaltender, insbesondere den Duftstoff in einer Verdünnungsflüssigkeit enthaltender Vorratsbehälter (Duftstoffspeicher 30), z. B. ein Kunststoff-, Metall- oder Glashohlkörper mit einer Entleerungsöffnung 31 befindet sich in Kopfüber-Stellung in einem Vorratsraum 13 in einem geeigneten Haltegestell. Der Vorratsraum kann, insbesondere in einem durch den Vorratsraumboden 13 A und den Flaschenhals des Duftstoffspeichers 30 verbleibenden Freiraum Schaltelemente 32 und andere etwa noch erforderliche, insbesondere elektrische und/oder elektronische Bauteile oder Gegenstände schützend aufnehmen.

Am unteren Ende des Vorratsraumbodens 13 A befindet sich ein kreisringförmiger, auf seiner Außenfläche gegebenenfalls kegelförmig gestalteter hohler Ventilhalter 33, der in dem dargestellten und insoweit bevorzugten Ausführungsbeispiel gleichzeitig, d.h. indirekt, als Schraubhalterung für den Duftstoffspeicher 30 dient.

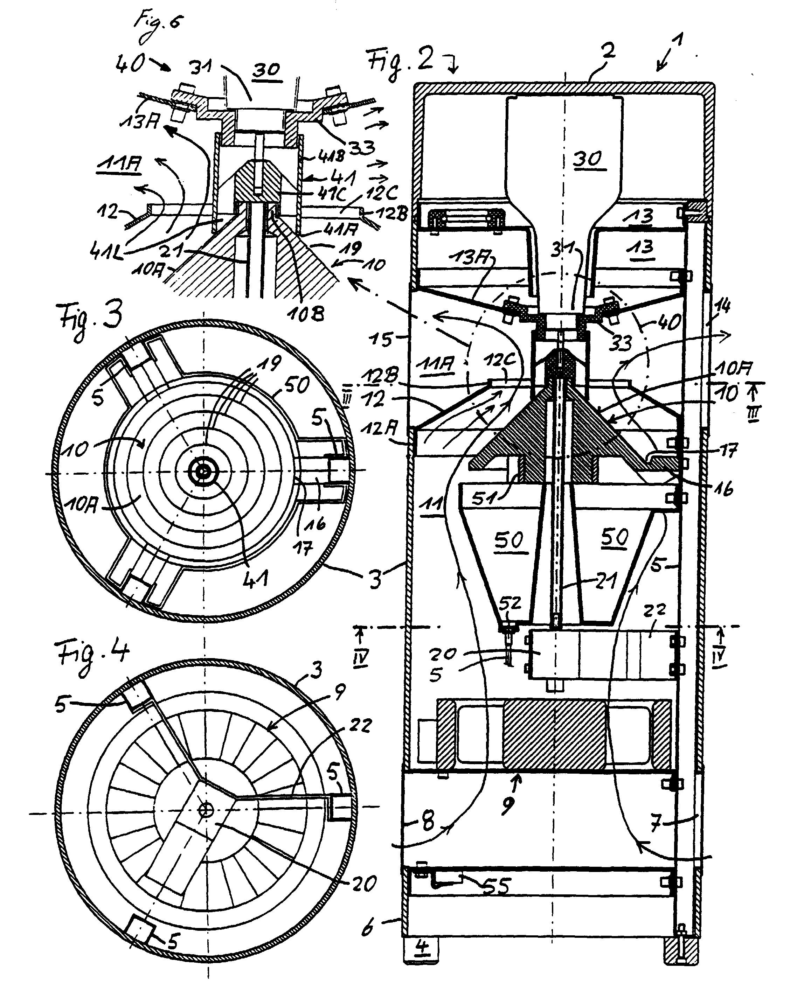

Damit die den Duftstoff beinhaltende Flüssigkeit aus dem Vorratsbehälter bei Bedarf dosiert abgezogen werden kann, ist ein insgesamt mit 40 beziffertes Dosierventil vorgesehen. Dieses besteht zum einen aus einem Dosierventilstößel 41 und zum anderen aus einer Dosiermimik 42. Einzelheiten des Dosierventiles und seine Funktion werden im Zusammenhang mit dem zweiten Ausführungsbeispiel nach Fign. 2 bis 7 noch näher erläutert werden. Jedenfalls ist der Dosierventilstößel 41 als zylindrischer, zum Teil hohler Körper gestaltet und derart angeordnet, daß er auf seiner radialen Außenseite als Luftleitfläche im Zusammenwirken mit dem Vorratsraumboden 13 A und dem Luftleitmantel 12 dazu dient, den bereits bedufteten Luftstrom nach radial außen umzulenken und dabei die radial zuinnerst liegende Wand des Radialströmungskanals 11 A zu bilden. Die den Duftstoff enthaltene Flüssigkeit wird bei jeden Dosierschritt durch innere Hohlräume des Dosierventilstößels 41 durch Schwerkraft nach unten geleitet und tritt am unteren schürzenförmigen Mündungsrand 41 A des Dosierventilstößels 41 auf die Flüssigkeitsverdunstungsfläche 10 A des Verdunstungskörpers 10 im Bereich von dessen Spitze über.

Die sich vom Mündungsrand 41 A des Dosierventilstößels 41 ausgehend auf die unter etwa 45° schräg nach unten geneigte Flüssigkeitsverdunstungsfläche 10 A sich durch Gravitation fein verteilende den Duftstoff enthaltende Flüssigkeit bildet einen sehr dünnen Feuchtigkeitsfilm auf dem Verdunstungskörper 10, der allenfalls den radialen Außenrand des Verdunstungskörpers 10 noch erreicht, dann aber (wenn er den Rand überhaupt erreicht) so dünn ist, daß Flüssigkeit nicht nach unten abtropfen kann. Lediglich für den Fall eines etwaigen Defektes befindet sich der nach oben konisch auseinanderlaufende Auffangbehälter 50 derart unterhalb des Verdunstungskörpers 10, daß am radialen Außenrand des Verdunstungskörpers 10 im Defektfalle abtropfende Flüssigkeit in den Auffangbehälter 50 hineinläuft. Hierzu ist dessen Fassungsvermögen größer oder zumindest gleich dem Fassungsvermögen des Duftstoffspeichers 30.

Da sich die zu beduftende Luft in der Beschleunigungszone des Strömungsspaltes 11, d.h. im Bereich der radialen Außenfläche des Verdunstungskörpers abkühlt, sind Heizelemente 51 mit dem Verdunstungskörper 10, z. B. wärmeleitend, derart verbunden, daß die erzielte Raumtemperatur und der Verdunstungsgrad nicht beeinträchtigt werden.

Ein Hubmittel 20, vorzugsweise ein Elektromagnet, ist in dem Abstandsspalt zwischen dem Gebläse 9 und dem Auffangbehälter 50 derart angeordnet, daß ein Hubstab 21 in zentraler Lage durch die Mitte des zu diesem Zweck ringbeckenförmig gestalteten Auffangbehälters 50 und die zentrisch durchbrochene Spitze des Verdunstungskörpers 10 zum Dosierventilstößel 41 geführt werden kann, um diesen für die Dosierphasen, insbesondere taktweise, heben und wieder senken zu können.

Dieses Raumbeduftungsgerät, welches in seinem hohlraumförmigen Sockel 6 auch elektrische und elektronische Bauteile für den Betrieb, d.h. auch die Steuerung aufnehmen kann, ist sehr servicefreundlich, weil der Duftspeicher 30 bei einem Wechseln mit aufgesetzter Dosiermimik 4 einfach in den Ventilhalter 33 eingeschraubt bzw. herausgeschraubt werden kann und alle gelegentlich zu wartende oder kontrollierende Bauteile völlig freigelegt werden können, weil das Gehäuse 3 aus rohrabschnittsförmigen Ringen besteht, die mit wenigen Schrauben an den Befestigungsstreben 5 lösbar befestigt sind und nach oben oder unten völlig abgezogen werden können. Das gleiche gilt im Bedarfsfall auch für die Loch- oder Gitterabdeckungen 8 und 15.

Das Ausführungsbeispiel nach Fign. 2 bis 7 unterscheidet sich von seinem grundsätzlichen Aufbau her von der Ausführungsform nach Fig. 1 praktisch gar nicht. Deshalb werden nachfolgend lediglich die besonderen Unterscheidungsmerkmale erläutert:

So ist eine Ablaßöffnung 52 des Auffangbehälters 50, die gemäß Fig. 1 mit einem Stopfen 53 versehen ist nach dem Ausführungsbeispiel der Fig. 2 mit einer Schlauchleitung 54 verbunden, welche in den Sockel 6 führt und dort über ein leicht zugängliches Schlauchventil 55 zum Entleeren des Auffangbehälters geöffnet und nachfolgend geschlossen werden kann.

Der wiederum kegelförmige Verdunstungskörper 10 ist lediglich zentrisch durchbohrt, im übrigen aber aus Vollmaterial, insbesondere als Metallgußteil hergestellt. Dadurch wird seine Wärmekapazität erhöht. Das Heizelement 51 ist hier ringförmig im unteren Bereich des Verdunstungskörpers mit diesem flächig verbunden und sorgt für eine Verdunstungskörpertemperatur von etwa 50°C. Der Verdunstungskörper 10 weist im übrigen drei nach radial außen weisende Tragarme 16 auf, die zum Ableiten von Flüssigkeit bei einem Gerätedefekt eine Sammelrinne 17 oder (wie in Fig. 1) eine Ablaßbohrung 18 aufweisen, so daß an dieser Stelle ablaufende Flüssigkeit sicher in den Auffangbehälter 50 gelangt. Durch die Tragarme 16 ist der bei diesen beiden Ausführungsbeispielen wegen der besseren Wärmespeicherung voluminös gestaltete Verdunstungskörper 10 solide an den Befestigungsstreben 5 festlegbar. Sollte der Verdunstungskörper jedoch dünnwandig und/oder sehr leichtgewichtig ausgestaltet sein, ist es auch denkbar, diesen ggf. gemeinsam mit dem Auffangbehälter, sozusagen als Teil des Dosierventilstößels zu heben und zu senken, um das Dosierventil zu betätigen. In der Regel wird aber die starre Ausführungsform bevorzugt.

Wie sich bei fortgelassenem Luftleitmantel aus der Fig. 3 ersehen läßt, ist der Verdunstungskörper 10 auf seiner radial außenliegenden Flüssigkeitsverdunstungsfläche 10 A mit kreisförmigen, etwa horizontalen Rillen 19 versehen. Hierdurch wird verhindert, daß sich einzelne Flüssigkeitssträhnen entlang der Schrägfläche nach unten schlängeln können. Die Rillen sorgen nämlich dafür, daß dort von oben her ankommende Flüssigkeit zunächst gespeichert wird und sich dabei auf dem Umfang gleichmäßig verteilt. Erst wenn dieser "Zwischenspeicher" voll ist, kriecht die Flüssigkeit - wie bei einem Überlauf - über den Rillenrand nach unten weiter und benetzt den nachfolgenden Teil der Flüssigkeitsverdunstungsfläche 10 A auf dem gesamten Umfang sehr gleichmäßig. Eine fein zerklüftete Oberfläche wie zum Beispiel eine gußrauhe Metalloberfläche erhöhen die Feinverteilung der Flüssigkeit vorteilhaft. Es ist auch denkbar, die Schrägfläche porös zu gestalten und dadurch einen zusätzlichen gewissen Speichereffekt für Flüssigkeit zu erzielen, ohne daß hierdurch die Gefahr besteht, daß die schnell vorbeistreichende Luft Flüssigkeitströpfchen mitreißt. Z. B. ist auch ein poröser Belag, wie z. B. aus filzähnlichem Material oder ein poröser Körper für die Feuchtigkeitsverdunstungsfläche denkbar.

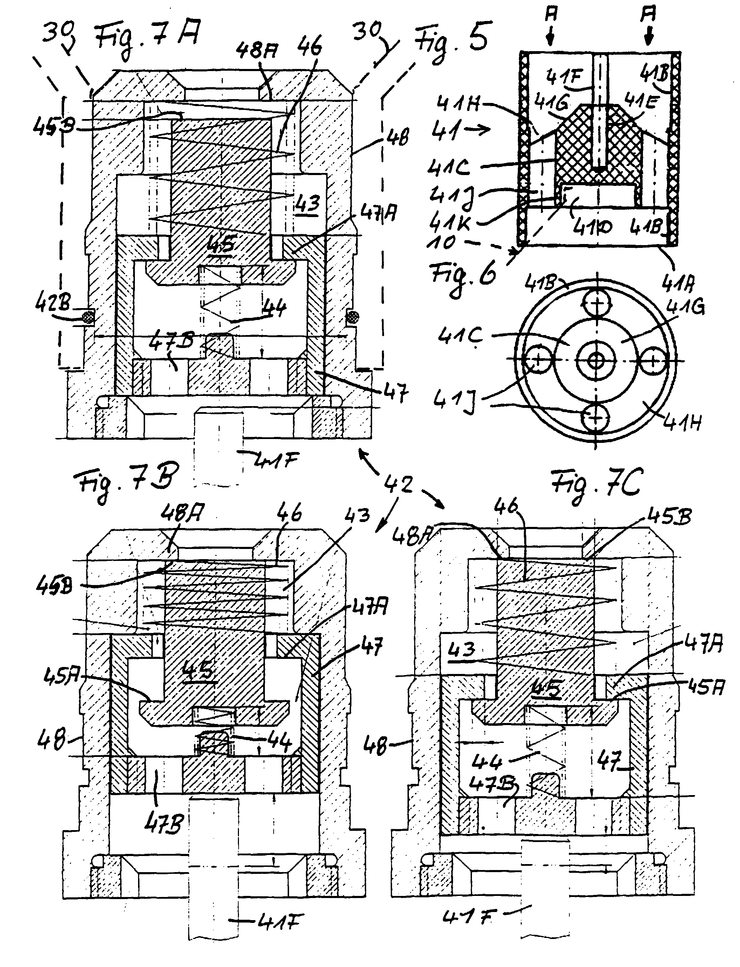

Die Arbeitsweise des Dosierventils wird nachfolgend anhand der Fign. 5 bis 7 näher erläutert:

Der Dosierventilstößel 41 besteht, wie sich aus Fign. 5 und 6 ergibt, aus einem kreiszylindrischen, zum Teil rohrförmig hohlen Bauteil mit Rohrwänden 41 B und einem Mittelteil 41 C mit einer unteren zentralen Aufnahme 41 D für das obere Ende des Hubstabes 21 einerseits und einen zylindrischen Ringkragen 10 B des Verdunstungskörpers 10 andererseits. Die Tiefe der Aufnahme reicht aus, um in allen Ventilstellungen eine axiale Überdeckung zwischen dem Mittelteil 41 C und dem Ringkragen 10 B aufrechtzuerhalten. Eine zentrische, nach oben offene Sackbohrung 41 E nimmt eine, vorzugsweise längenkalibrierte, Stößelverlängerung 41 F auf, welche die nachfolgende Dosiermimik 42 betätigt. Dies wird im Zusammenhang mit Fign. 7 A bis C beschrieben werden.

Die obere Rohrwand 41 B umfaßt gleitverschieblich das untere kreiszylindrische Ende des Ventilhalters 33, so daß sich der Dosierventilstößel 41 heben und senken kann, ohne daß Luft in sein Inneres in nennenswertem Umfang eintreten kann. Dadurch ist der Flüssigkeitstransport innerhalb des Dosierventilstößels 41 von der Luftströmung unbeeinflußt.

Die von oben her aus der Dosiermimik 42 austretende Flüssigkeit wird daher zwischen dem Mittelteil 41 C und der oberen Rohrwand 41 B aufgefangen und über eine kegelstumpfförmige Leitfläche 41 G in eine Sammelrinne 41 H gleichmäßig umfangsverteilt. Vom Boden der Sammelrinne 41 H das Mittelteil 41 C nach unten gleichmäßig umfangsverteilt durchdringende Führungskanäle 41 J leiten die Flüssigkeit an der Schürze 41 K der Aufnahme 41 D außen vorbei unmittelbar auf die Oberfläche des Verdunstungskörpers 10. Die Länge der Schürze 41 K sowie die Länge der unteren Rohrwand 41 B sind derart aufeinander abgestimmt, daß beide Ringränder bei gesenktem Dosierventilstößel, vorzugsweise scharfkantig, also wie bei einer Abrißkante, auf der Flüssigkeitsverdunstungsfläche 10 A aufsitzen. Dadurch bildet sich zum einen ein ringförmiger Flüssigkeitssammelraum 41 L und andererseits werden durch körperliche Berührung die Adhäsionskräfte der Flüssigkeit am Mündungsrand 41 A und am unteren Rand der Schürze 41 K unmittelbar auf die Flüssigkeitsverdunstungsfläche 10 A übergeleitet, so daß ein gleichmäßig umfangsverteilter Flüssigkeitsfilm sich von vornherein auf dem Verdunstungskörper 10 ausbilden kann.

Aus Fign. 7 A bis C ist die Dosiermimik 42 im einzelnen zu erkennen. Ein zylindrisches Ventilgehäuse 48 ist über einen O-Ring 42 B dichtend und unverlierbar, möglichst sogar nicht entfernbar (Originalitätsverschluß) in die Entleerungsöffnung 31 eines Duftstoffspeichers 30 eingesetzt. Im Gehäuseinneren befinden sich eine Dosierkammer 43, ein mit einer Druckfeder 44 belasteter Dosierstößel 45 sowie ein mit einer Druckfeder 46 belasteter hohler Steuerstößel 47. In der Darstellung nach Fig. 7 A ist das Dosierventil geschlossen, weil sich der Dosierstößel 45 mit seinem unteren flanschartigen Rand 45 A unter dem Druck der Federn 44 und 46 an dem oberen flanschartigen Rand 45 A des Steuerstößels 47 dichtend abstützt. Bei einem Anheben der Hubstabes 21 durch den Elektromagneten 20, wird der Dosierventilstößel 41 vom Verdunstungskörper 10 zunächst leicht abgehoben und schiebt die Stößelverlängerung 41 F den Steuerstößel 47 nach oben, so daß sich der Dosierstößel 45 mit einer oberen Ringdichtfläche 45 B an einem Dichtflansch 48 A des Ventilgehäuses 48 unter dem Druck der Feder 44 dichtend abstützt. Dadurch verschließt sich zunächst die Dosierkammer 43, welche nunmehr ein wohldefiniertes Flüssigkeitsvolumen einschließt (Fig. 7 B). Ein weiteres Heben des Steuerstößels 47 öffnet die Dichtstelle 45 A / 47 A und läßt das eingesperrte Flüssigkeitsvolumen durch Auslaßöffnungen 47 B durch Gravitation in den oberen Hohlraum des Dosierventilstößels 41 übertreten.

Dadurch daß in dieser Ventilposition (Fig. 7 C) der Dosierventilstößel 41 von der Flüssigkeitsverdunstungsfläche 10 A abgehoben ist, kann eine ausreichende Luftmenge durch den Dosierventilstößel 41 in das Innere des Steuerstößels 47 und die Dosierkammer 43 eintreten, so daß bei nachfolgendem Schließen des Ventils zunächst die gesamte Dosierkammer 43 mit Luft gefüllt ist (entsprechend Fig. 7 B) und nachfolgend diese Luft in den Duftstoffspeicher 30 übertreten kann (entsprechend Fig. 7 A). Dadurch wird trotz sehr feiner Dosierbarkeit die Ausbildung eines Vakuums im Duftstoffspeicher 30 vermieden. Die Arbeitsdauer des Dosierventils, z. B. während eines Tages, sowie die Häufigkeit der Dosiertakte je Zeiteinheit, z. B. während einer Stunde, und gegebenenfalls andere Betriebsgrößen des Raumbeduftungsgerätes sind vorzugsweise frei vorgebbar, insbesondere programmierbar, so daß ein automatischer, gegebenenfalls auch geregelter Dosierbetrieb möcht licht. In jedem Falle erfolgt die Regelung oder Steuerung der Duftintensität durch die Taktungsfrequenz des Dosierventils, d. h. über die Zugabemenge an Duftstoff je Zeiteinheit an die Schrägfläche. Die Stärke des zu bedufteten Luftstromes, d. h. seine Volumenrate, wird so eingestellt, daß ein Abtropfen von Duftstoff enthaltender Flüssigkeit am unteren Rand der Schrägfläche vermieden wird.

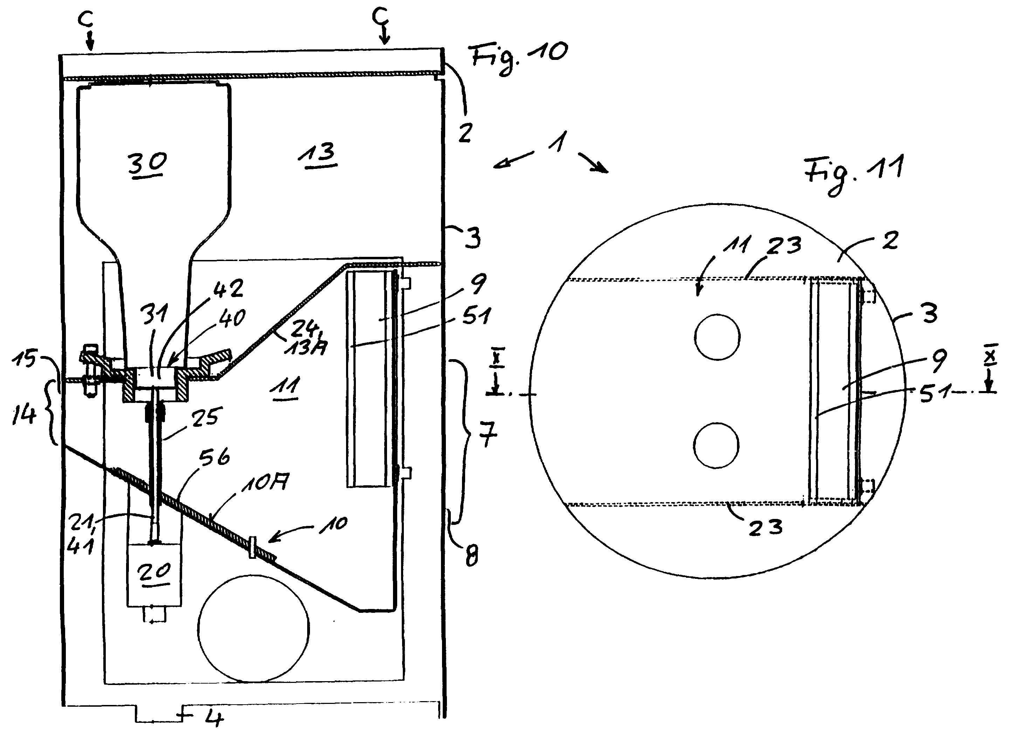

Eine vergleichbare Dosierventilbetätigung ist bei dem vierten Ausführungsbeispiel nach Fign. 10 und 11 vorgesehen. Auch hier ist ein zylindrisches Gehäuse 3 mit Lufteinlaß 7 und Luftauslaß 14 vorgesehen. Ein hinter dem Lufteinlaß 7 mit horizontal liegender Achse angeordnetes Gebläse 9 leitet die Luft in einen von senkrechten Seitenwänden 23 begrenzten Strömungskanal mit sich in Strömungsrichtung schräg absenkender Deckenwand 24 und schräg aufsteigender Flüssigkeitsverdunstungsfläche 10 A. Im Bereich des engsten Querschnitts des Strömungsspaltes 11 ist wiederum kopfüber der Duftspeicher 30 nach Art der Fig. 2 angeordnet. Ein Schutzrohr 25 ist dichtend durch die Flüssigkeitsverdunstungsfläche 10 A nach oben geführt um in ihrem Inneren den Hubstab 21 aufzunehmen. Als Heizelement 51 für die Luft im Strömungsspalt 11 und die Flüssigkeitsverdunstungsfläche 10 A dient eine Glühbirne oder dergleichen. Ansonsten entspricht die Funktion den Ausführungsbeispielen nach Fig. 1 und 2.

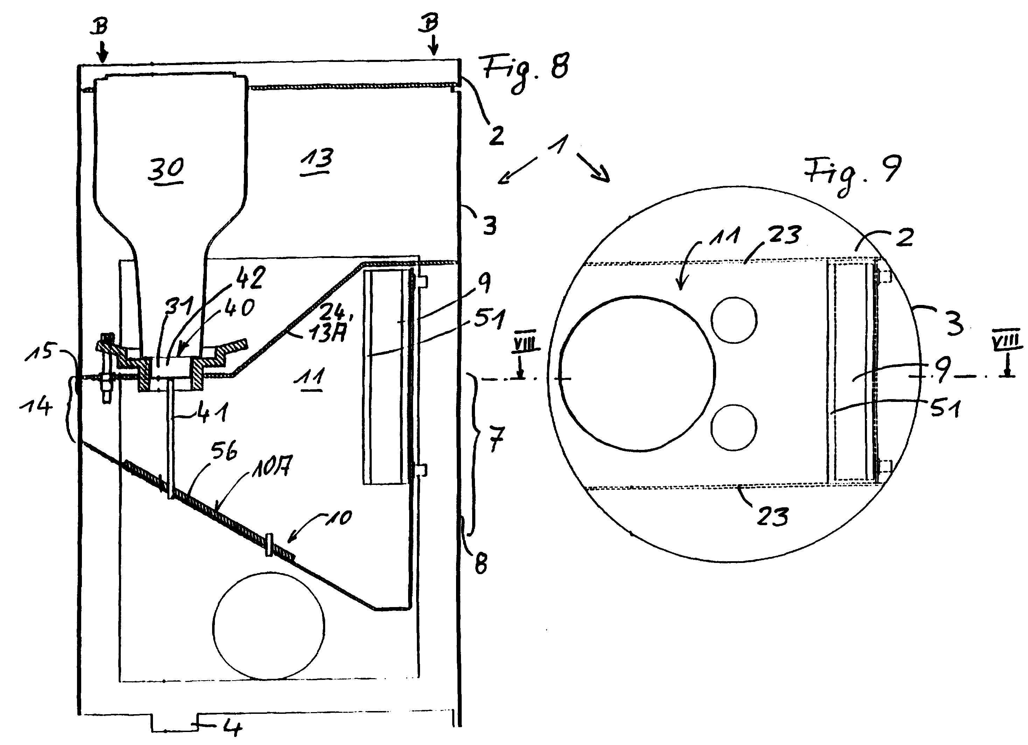

Das in Fign. 8 und 9 dargestellte dritte Ausführungsbeispiel stellt eine vereinfachte Version zu dem Ausführungsbeispiel nach Fign. 10 und 11 dar. Dieses Gerät ist für eine Dosierung per Hand vorgesehen. Zu diesem Zweck ist ein starr mit dem Verdunstungskörper 10 verbundener Dosierventilstößel 41 vorgesehen, der an einem im einzelnen nicht dargestellten Dosierventil im Bereich der Entleerungsöffnung 31 des Duftstoffspeichers 30 anliegt. Der Duftstoffspeicher 30 ist aus elastischem Material oder weist zumindest einen elastischen (in Gebrauchsstellung oben liegenden) Boden auf oder ist insgesamt durch Druck von oben mit einer Hand nach unten geringfügig reversibel verlagerbar, so daß jede dieser Pump-Aktionen die gleiche Wirkung wie das Betätigen eines Hubstabes 21 hat.

Wie sich aus Fig. 12 ergibt, kann ein Raumbeduftungsgerät nach einem der vorangehenden Ausführungsbeispiele über einen Luftauslaßstutzen 14 A, der sich an einen Radialströmungskanal 11 A anschließt mit einem Lüftungskanal einer stationären Lüftungsanlage fluidisch verbunden werden.

Schließlich ist es auch möglich, Raumbeduftungsgeräte, etwa nach Art der Fign. 8 bis 11 in der Weise weiter zu vereinfachen, daß als Gehäuse und gleichzeitig als Luftleitkanal z. B. ein horizontal angeordnetes Rechteckrohr verwendet wird, an dessen einem Mündungsende ein Gebläse mit integrierten oder nachgeordneten Heizelementen und an dessen anderem Mündungsende der Luftauslaß vorgesehen ist. Im Bereich des hinteren Endes dieses Strömungskanals ist wiederum ein Duftstoffspeicher etwa nach Art des dritten und vierten Ausführungsbeispieles angeordnet. Als Schrägfläche für die Verteilung und Flüssigkeitsverdunstung dient ein keilförmiges Bauteil, das von oben mit Duftstoff enthaltender Flüssigkeit dosiert betropft wird und von der Luftströmung überstrichen wird. Eine besonders hohe Speicherfähigkeit kann durch Verwendung eines Verdunstungskörpers aus porösem Material, wie Gasbeton oder dergleichen, erzielt werden.

Bei allen Ausführungsbeispielen wird die Flüssigkeitsverdunstungsfläche von ihrem unten gelegenen Ende her von der zu beduftenden Luft angeströmt und erfolgt die Flüssigkeitszuführung im Bereich des oben liegenden Endes dieser Schrägfläche; damit wird in besonders vorteilhafter Weise eine Gegenstromverdunstung realisiert. Während aus Gravitationsgründen die Flüssigkeitszuführung im oberen Endbereich obligatorisch ist, kann die Luftbeduftung auch im Gleichstrom oder im Querstrom zur Flüssigkeitsausbreitung auf der Flüssigkeitsverdunstungsfläche erfolgen.

### Bezugszeichenliste

- 1: Raumbeduftungsgerät
- 2: Haube
- 3: Gehäuse
- 4: Standfüße
- 5: Befestigungsstreben
- 6: Sockel
- 7: Lufteinlaß
- 8: Loch- oder Gitterabdeckungen
- 9: Gebläse
- 10: Verdunstungskörper
- 10 A: Flüssigkeitsverdunstungsfläche
- 10 B: Ringkragen
- 11: Strömungsspalt
- 11 A: Radialströmungskanal
- 12: Luftleitmantel
- 12 A: Schürze
- 12 B: Schürze
- 12 C: Luftabströmöffnung
- 13: Vorratsraum
- 13 A: Vorratsraumboden
- 14: Luftauslaß
- 14 A: Luftauslaßstutzen
- 14 B: Ringkanalabdeckung
- 15: Loch- oder Gitterabdeckung
- 16: Tragarme
- 17: Sammelrinne
- 18: Ablaßbohrung
- 19: Rillen
- 20: Hubmittel
- 21: Hubstab
- 22: Hubmittelhalter
- 23: Seitenwände
- 24: Deckenwand
- 25: Schutzrohr
- 30: Duftstoffspeicher
- 31: Entleerungsöffnung
- 32: Schaltelemente
- 33: Ventilhalter
- 40: Dosierventil
- 41: Dosierventilstößel
- 41 A: Mündungsrand
- 41 B: Rohrwände
- 41 C: Mittelteil
- 41 D: Aufnahme
- 41 E: Sackbohrung
- 41 F: Stößelverlängerung
- 41 G: Leitfläche
- 41 H: Sammelrinne
- 41 J: Führungskanäle
- 41 K: Schürze
- 41 L: Flüssigkeitssammelraum
- 42: Dosiermimik
- 42 B: O-Ring
- 43: Dosierkammer
- 44: Druckfeder
- 45: Dosierstößel
- 45 A: Dichtflansch
- 45 B: Ringdichtfläche
- 46: Druckfeder
- 47: Steuerstößel
- 47 A: Dichtflansch
- 47 B: Auslaßöffnungen
- 48: Ventilgehäuse
- 48 A: Dichtflansch
- 50: Auffangbehälter
- 51: Heizelemente
- 52: Ablaßöffnungen
- 53: Stopfen
- 54: Schlauchleitung
- 55: Schlauchventil
- 56: Vlies

## Patentansprüche

1. Raumbeduftungsgerät,
- mit einem Luftleitkanal,
- mit einem Gebläse zum Fördern von zu beduftender Luft durch den Luftleitkanal,
- mit einem Vorratsbehälter für einen einen Duftstoff beinhaltenden Flüssigkeitsvorrat und
- mit einer Flüssigkeitsverdunstungsfläche, über die der zu beduftende Luftstrom zur Aufnahme von verdunstendem Duftstoff geleitet wird,
**dadurch gekennzeichnet**,
daß der Vorratsbehälter eine nach unten weisende Entleerungsöffnung (31) aufweist, durch welche die Flüssigkeit den Vorratsbehälter nach unten verläßt, um sich auf eine unter der Entleerungsöffnung gelegene, bezüglich der Entleerungsöffnung schräg nach unten geneigte, als Flüssigkeitsverdunstungsfläche (10 A) dienende Schrägfläche eines Verdunstungskörpers (10) zu verteilen,
daß ein die Entleerungsöffnung beherrschendes Dosierventil (40) zur dosierten Abgabe von Flüssigkeit aus dem Vorrat an die Schrägfläche vorgesehen ist und
daß die Schrägfläche innerhalb des Luftleitkanals derart angeordnet ist, daß der zu beduftende Luftstrom intensiv über die Schrägfläche streicht.

2. Raumbeduftungsgerät nach Anspruch 1, dadurch gekennzeichnet, daß der Verdunstungskörper (10) kegelförmig oder hohlkegelförmig gestaltet ist.

3. Raumbeduftungsgerät nach Anspruch 2, dadurch gekennzeichnet, daß der Verdunstungskörper (10) nach unten durch einen Verdrängungskörper (50) verlängert ist.

4. Raumbeduftungsgerät nach einem der Ansprüche 1 bis 3, dadurch gekennzeichnet, daß die Flüssigkeitsverdunstungsfläche (10 A) zur Oberflächenvergrößerung rauh, profiliert oder porös ausgebildet ist.

5. Raumbeduftungsgerät nach einem der Ansprüche 1 bis 4, dadurch gekennzeichnet, daß die Flüssigkeitsverdunstungsfläche (10 A) mit etwa horizontalen Flüssigkeitssammel-/Überlaufrillen (19) versehen ist.

6. Raumbeduftungsgerät nach einem der Ansprüche 1 bis 5, dadurch gekennzeichnet, daß der Verdunstungskörper aus Aluminium, Keramikmaterial und/oder porösem Material besteht oder mit einem porösen Flächenmaterial, wie einem Vlies, aus Papier, Kunststoff, Textil- oder Keramikmaterial belegt ist.

7. Raumbeduftungsgerät nach einem der Ansprüche 1 bis 6, gekennzeichnet durch einen an den unteren Rand der Flüssigkeitsverdunstungsfläche (10 A) nach unten sich anschließenden Flüssigkeitsauffangbehälter (50).

8. Raumbeduftungsgerät nach einem der Ansprüche 1 bis 7, dadurch gekennzeichnet, daß das Dosierventil (40) einen Dosierventilstößel (41) aufweist, der von einer durch die Flüssigkeitsverdunstungsfläche (10 A) hindurch geführten Hubstange (21) heb- und senkbar ist.

9. Raumbeduftungsgerät nach einem der Ansprüche 1 bis 8, dadurch gekennzeichnet, daß das Dosierventil (40) einen etwa zylindrischen Dosierventilstößel (41) aufweist, dessen radiale Außenseite als Luftleitfläche dient und welcher mit mindestens einem inneren Führungskanal (41 J) zum Überführen von Flüssigkeit aus dem Vorratsbehälter auf die Flüssigkeitsverdunstungsfläche (10 A) versehen ist.

10. Raumbeduftungsgerät nach Anspruch 8 oder 9, dadurch gekennzeichnet, daß der Dosierventilstößel (41) bei geschlossenem Ventil, insbesondere mit einer Kante, auf der Flüssigkeitsverdunstungsfläche (10 A) aufsitzt.

11. Raumbeduftungsgerät nach einem der Ansprüche 1 bis 10, dadurch gekennzeichnet, daß das Dosierventil (40) eine schleusenartig betätigbare Dosierkammer (43) aufweist.

12. Raumbeduftungsgerät nach Anspruch 11, gekennzeichnet durch einen federdruckbelasteten Dosierstößel (45) und einen federdruckbelasteten Steuerstößel (47) innerhalb eines Ventilgehäuses (48) zum schleusenartigen Befüllen und Entleeren der Dosierkammer (43).

13. Raumbeduftungsgerät nach einem der Ansprüche 1 bis 12, dadurch gekennzeichnet, daß der Vorratsbehälter, z. B. in Gestalt einer Auswechselkassette oder einer Auswechselflasche (30), kopfüber oberhalb der Flüssigkeitsverdunstungsfläche (10 A) angeordnet ist.

14. Raumbeduftungsgerät nach einem der Ansprüche 1 bis 13, dadurch gekennzeichnet, daß in die Entleerungsöffnung (31) des Vorratsbehälters die Dosiermimik (42) des Dosierventils (41) festsitzend integriert ist.

15. Raumbeduftungsgerät nach einem der Ansprüche 1 bis 14, dadurch gekennzeichnet, daß der Luftströmungskanal im Bereich der Flüssigkeitsverdunstungsfläche (10 A) einen sich in Strömungsrichtung der Luft verjüngenden Querschnitt aufweist.

16. Raumbeduftungsgerät nach einem der Ansprüche 1 bis 15, dadurch gekennzeichnet, daß der Luftströmungskanal sich nach dem Ende der Flüssigkeitsverdunstungsfläche (10 A), gegebenenfalls unter Umlenkung, querschnittserweitert.

17. Raumbeduftungsgerät nach Anspruch 15 oder 16, gekennzeichnet durch einen kegelstumpfförmigen Luftleitmantel (12).

18. Raumbeduftungsgerät nach einem der Ansprüche 1 bis 17, dadurch gekennzeichnet, daß der Luftleitkanal ringförmig, insbesondere kreisringförmig, gestaltet ist.

19. Raumbeduftungsgerät nach Anspruch 18, gekennzeichnet durch eine einen Luftauslaß (14) verschließende, einen Luftauslaßstutzen (14 A) aufweisende Ringkanalabdeckung (14 B), insbesondere zur Verbindung mit einer, z. B. gebäudeeigenen, Luftkanalisation.

20. Raumbeduftungsgerät nach einem der Ansprüche 1 bis 19, gekennzeichnet durch einen Anschluß des Luftauslasses des Raumbeduftungsgerätes an eine gebäudeeigene oder zusätzlich verlegte Luftkanalisation.

21. Verfahren zum Beduften von Räumen, bei dem die zu beduftende Luft mittels eines Gebläses durch einen Luftleitkanal über eine mit einer einen Duftstoff beinhaltenden Flüssigkeit beaufschlagte Flüssigkeitsverdunstungsfläche geleitet wird,
**dadurch gekennzeichnet,**
daß ein Vorratsbehälter für die den Duftstoff beinhaltende Flüssigkeit verwendet wird, der eine nach unten wirksame Entleerungsöffnung aufweist, durch welche die Flüssigkeit den Vorratsbehälter nach unten verläßt, um sich auf eine unter der Entleerungsöffnung gelegene, bezüglich der Entleerungsöffnung schräg nach unten geneigte, als Flüssigkeitsverdunstungsfläche dienende Schrägfläche eines Verdunstungskörpers zu verteilen,
daß die Flüssigkeit aus dem Vorrat an die Schrägfläche dosiert abgegeben wird und
daß der zu beduftende Luftstrom intensiv derart über die Schrägfläche streicht, daß dabei am unteren Ende der Schrägfläche praktisch keine Flüssigkeit mehr anfällt oder überschüssig ist.

22. Verfahren nach Anspruch 21, dadurch gekennzeichnet, daß der Verdunstungskörper und/oder der zu beduftende Luftstrom beheizt wird.

23. Verfahren nach Anspruch 21 oder 22, dadurch gekennzeichnet, daß die Beduftungsintensität über die je Zeiteinheit der Schrägfläche zudosierte Flüssigkeitsmenge, vorzugsweise in frei programmierbarer Weise, gesteuert oder geregelt wird.

## Claims

1. A room fragrancing apparatus,
- having an air guiding channel,
- having a fan for conveying, through the air guiding channel, air to be fragranced,
- having a storage container for a fluid store containing an odorous substance and
- having a fluid evaporation surface over which the air stream to be fragranced is passed so as to receive evaporating odorous substance,
characterised
in that the storage container has a downwardly directed discharge opening (31) through which the fluid leaves the storage container in the downwards direction in order to distribute itself on an inclined surface - of an evaporation body (10) - which is located below the discharge opening, is downwardly inclined with respect to the discharge opening and serves as a fluid evaporation surface (10A),
in that a metering valve (40) controlling the discharge opening is provided for metered discharge of fluid from the store to the inclined surface and
in that the inclined surface is arranged within the air guiding channel in such a manner that the air stream to be fragranced brushes over the inclined surface in an intensive manner.

2. A room fragrancing apparatus in accordance with Claim 1, characterised in that the evaporation body (10) is cone-shaped or hollow-cone-shaped.

3. A room fragrancing apparatus in accordance with Claim 2, characterised in that the evaporation body (10) is extended downwards by a displacement body (50).

4. A room fragrancing apparatus in accordance with any one of Claims 1 to 3, characterised in that the fluid evaporation surface (10A) is rough, profiled or porous to enlarge the surface.

5. A room fragrancing apparatus in accordance with any one of Claims 1 to 4, characterised in that the fluid evaporation surface (10A) is provided with substantially horizontal fluid collection/overflow grooves (19).

6. A room fragrancing apparatus in accordance with any one of Claims 1 to 5, characterised in that the evaporation body is composed of aluminium, ceramic material and/or porous material or is covered with a porous surface-material, such as a non-woven fabric, made of paper, a plastics material, a textile or ceramic material.

7. A room fragrancing apparatus in accordance with any one of Claims 1 to 6, characterised by a fluid catching container (50) downwardly adjacent to the lower edge of the fluid evaporation surface (10A).

8. A room fragrancing apparatus in accordance with any one of Claims 1 to 7, characterised in that the metering valve (40) has a metering valve tappet (41) which can be raised and lowered by a lifting rod (21) passed through the fluid evaporation surface (10A).

9. A room fragrancing apparatus in accordance with any one of Claims I to 8, characterised in that the metering valve (40) has a substantially cylindrical metering valve tappet (41) whose radial outer surface serves as an air guiding surface and which is provided with at least one inner guide channel (41 J) for conveying fluid from the storage container to the fluid evaporation surface (10A).

10. A room fragrancing apparatus in accordance with Claim 8 or 9, characterised in that the metering valve tappet (41) rests, in particular by means of one edge, on the fluid evaporation surface (10A) when the valve is closed.

11. A room fragrancing apparatus in accordance with any one of Claims 1 to 10, characterised in that the metering valve (40) has a metering chamber (43) operable in a sluice-like manner.

12. A room fragrancing apparatus in accordance with Claim 11, characterised by a spring-loaded metering tappet (45) and a spring-loaded control tappet (47) within a valve housing (48) for sluice-like filling and emptying of the metering chamber (43).

13. A room fragrancing apparatus in accordance with any one of Claims 1 to 12, characterised in that the storage container, e.g. in the form of an exchangeable container or an exchangeable bottle (30), is arranged upside-down above the fluid evaporation surface (10 A).

14. A room fragrancing apparatus in accordance with any one of Claims 1 to 13, characterised in that the dosing device (42) of the metering valve (41) is integrated in a tightly fitting manner into the discharge opening (31) of the storage container.

15. A room fragrancing apparatus in accordance with any one of Claims 1 to 14, characterised in that in the region of the fluid evaporation surface (10 A), the air flow channel tapers in cross-section in the direction of flow of the air.

16. A room fragrancing apparatus in accordance with any one of Claims 1 to 15, characterised in that the air flow channel widens in cross-section after the end of the fluid evaporation surface (10 A), possibly with deflection.

17. A room fragrancing apparatus in accordance with Claim 15 or 16, characterised by an air guiding cover (12) of truncated-cone shape.

18. A room fragrancing apparatus in accordance with any one of Claims 1 to 17, characterised in that the air guiding channel is ring-shaped, in particular circular-ringshaped.

19. A room fragrancing apparatus in accordance with Claim 18, characterised by an annular channel covering (14 B) which closes an air outlet (14), has an air outlet connection (14 A) and is in particular for connection to air channelling which is for example integral to the building.

20. A room fragrancing apparatus in accordance with any one of Claims 1 to 19, characterised by connection of the air outlet of the room fragrancing apparatus to air channelling which is integral to the building or additional.

21. A method of fragrancing rooms, in which, by means of a fan, the air to be fragranced is passed, through an air guiding channel, over a fluid evaporation surface acted upon by a fluid containing an odorous substance, characterised
in that a storage container is used for the fluid containing the odorous substance and has a discharge opening which opens in the downwards direction and through which the fluid leaves the storage container in the downwards direction in order to distribute itself on an inclined surface - of an evaporation body - which is located below the discharge opening, is downwardly inclined with respect to the discharge opening and serves as a fluid evaporation surface,
in that the fluid is discharged in a metered manner from the store to the inclined surface and
in that the air stream to be fragranced brushes intensively over the inclined surface such that as a result fluid virtually no longer occurs or is surplus at the lower end of the inclined surface.

22. A method in accordance with Claim 21, characterised in that the evaporation body and/or the air stream to be fragranced is/are heated.

23. A method in accordance with Claim 21 or 22, characterised in that the fragrancing intensity is controlled or regulated, preferably in a freely programmable manner, via the quantity of fluid supplied by metering to the inclined surface per unit of time.

## Revendications

1. Appareil pour odoriférer une pièce
- avec un canal de guidage d'air,
- avec une soufflerie pour convoyer l'air à odoriférer à travers le canal de guidage d'air,
- avec un réservoir de stockage pour une réserve de liquide contenant une matière odorante et
- avec une surface d'évaporation de liquide sur laquelle est guidé le flux d'air à odoriférer pour recevoir une matière odorante évaporée,
caractérisé en ce que le réservoir de stockage présente une ouverture de vidange (31) orientée vers le bas à travers laquelle le liquide quitte le réservoir de stockage vers le bas pour se répartir sur une face inclinée d'un corps d'évaporation (10) situé en dessous de l'ouverture de vidange, s'étendant relativement à l'ouverture de vidange en biais vers le bas, servant de surface d'évaporation de liquide (10A),
en ce qu'il est prévu une vanne de dosage (40) située dans l'ouverture de vidange, en vue d'une émission dosée de liquide du réservoir de stockage à la face inclinée et
en ce que la face inclinée est disposée à l'intérieur du canal de guidage d'air de telle façon que le flux d'air à odoriférer passe d'une manière intense sur la face inclinée.

2. Appareil pour odoriférer une pièce selon la revendication 1, caractérisé en ce que le corps d'évaporation (10) est réalisé en forme de cône ou en forme de cône creux.

3. Appareil pour odoriférer une pièce selon la revendication 2, caractérisé en ce que le corps d'évaporation (10) est prolongé vers le bas par un corps de refoulement (50).

4. Appareil pour odoriférer une pièce selon l'une des revendications 1 à 3, caractérisé en ce que la surface d'évaporation de liquide (10A), en vue d'un agrandissement de la surface, est réalisée d'une manière rugueuse, profilée ou poreuse.

5. Appareil pour odoriférer une pièce selon l'une des revendications 1 à 4, caractérisé en ce que la surface d'évaporation du liquide (10A) est pourvue de rainures de collecte/de débordement de liquide (19) à peu près horizontales.

6. Appareil pour odoriférer une pièce selon l'une des revendications 1 à 5, caractérisé en ce que le corps d'évaporation est réalisé en aluminium, en matière céramique et/ou en un matériau poreux ou est garni d'un matériau de surface poreux, comme d'une nappe, en papier, matière synthétique, matière textile ou céramique.

7. Appareil pour odoriférer une pièce selon l'une des revendications 1 à 6, caractérisé par un récipient de collecte de liquide (50) faisant suite au bord inférieur de la surface d'évaporation de liquide (10A) vers le bas.

8. Appareil pour odoriférer une pièce selon l'une des revendications 1 à 7, caractérisé en ce que la vanne de dosage (40) présente un poussoir de vanne de dosage (41) qui peut être remonté et abaissé par une tige de levage (21) guidée à travers la surface d'évaporation de liquide (10A).

9. Appareil pour odoriférer une pièce selon l'une des revendications 1 à 8, caractérisé en ce que la vanne de dosage (40) présente un poussoir de vanne de dosage (41) à peu près cylindrique dont le côté extérieur radial sert de surface de guidage d'air et qui est pourvue d'au moins un canal de guidage intérieur (41J) pour faire passer le liquide du réservoir de stockage sur la surface d'évaporation de liquide (10A).

10. Appareil pour odoriférer une pièce selon l'une des revendications 8 ou 9, caractérisé en ce que le poussoir de vanne de dosage (41), lorsque la vanne est fermée, repose notamment avec une arête sur la surface d'évaporation de liquide (10A).

11. Appareil pour odoriférer une pièce selon l'une des revendications 1 à 10, caractérisé en ce que la vanne de dosage (40) présente une chambre de dosage (43) actionnable à la manière d'une écluse.

12. Appareil pour odoriférer une pièce selon la revendication 11, caractérisé par un poussoir de dosage (45) soumis à la pression d'un ressort et par un poussoir de commande (47) soumis à la pression d'un ressort à l'intérieur d'une cage de soupape (48) pour le remplissage et la vidange à la manière d'une écluse de la chambre de dosage (43).

13. Appareil pour odoriférer une pièce selon l'une des revendications 1 à 12, caractérisé en ce que le réservoir de stockage, par exemple sous la forme d'une cassette échangeable ou d'une bouteille échangeable (30) est disposé la tête en bas au-dessus de la surface d'évaporation de liquide (10A).

14. Appareil pour odoriférer une pièce selon l'une des revendications 1 à 13, caractérisé en ce qu'il est intégré fixement dans l'ouverture de vidange (31) du réservoir de stockage, l'élément de dosage (42) de la vanné de dosage (41).

15. Appareil pour odoriférer une pièce selon l'une des revendications 1 à 14, caractérisé en ce que le canal de guidage d'air présente au voisinage de la surface d'évaporation du liquide (10A) une section transversale diminuant dans la direction d'écoulement de l'air.

16. Appareil pour odoriférer une pièce selon l'une des revendications 1 à 15, caractérisé en ce que le canal d'écoulement d'air s'élargit en section transversale vers l'extrémité de la surface d'évaporation de liquide (10A), le cas échéant par une déviation.

17. Appareil pour odoriférer une pièce selon la revendication 15 ou 16, caractérisé par un manteau de guidage d'air tronconique (12).

18. Appareil pour odoriférer une pièce selon l'une des revendications 1 à 17, caractérisé en ce que le canal de guidage d'air est réalisé en forme d'anneau, notamment en forme d'anneau de cercle

19. Appareil pour odoriférer une pièce selon la revendication 18, caractérisé par un recouvrement de canal annulaire (14B) fermant une sortie d'air (14) et présentant une tubulure de sortie d'air (14A), notamment pour l'assemblage avec une canalisation d'air, par exemple propre au bâtiment.

20. Appareil pour odoriférer une pièce selon l'une des revendications 1 à 19, caractérisé par un raccordement de la sortie d'air de l'appareil pour odoriférer une pièce à une canalisation d'air propre au bâtiment ou posée additionnellement.

21. Procédé pour odoriférer des pièces, où l'air à odoriférer est guidé par une soufflerie à travers un canal de guidage d'air sur une surface d'évaporation de liquide chargée par un liquide contenant une matière odorante,
**caractérisé en ce**
qu'il est utilisé un réservoir de stockage pour le liquide contenant la matière odorante qui présente une ouverture de vidange efficace vers le bas à travers laquelle le liquide quitte le réservoir de stockage vers le bas pour se répartir sur une face inclinée d'un corps d'évaporation, situé en dessous de l'ouverture de vidange, s'étendant relativement à l'ouverture de vidange en biais vers le bas et servant de surface d'évaporation de liquide,
en ce que le liquide est émis d'une manière dosée du réservoir de stockage à la face inclinée et
en ce que le flux d'air à rendre odorant passe d'une manière intense de telle manière sur la face inclinée qu'à l'extrémité inférieure de la face inclinée, il n'y a pratiquement plus de liquide ou d'excédent de liquide.

22. Procédé selon la revendication 21, caractérisé en ce que le corps d'évaporation et/ou le flux d'air à rendre odorant est chauffé.

23. Procédé selon la revendication 21 ou 22, caractérisé en ce que l'intensité de l'odorification est commandée ou réglée par la quantité de liquide amenée d'une manière dosée par unité de temps à la face inclinée, de préférence d'une manière librement programmable.
